(19) Europäisches Patentamt
European Patent Office
Office européen des brevets

(11) Publication number : **0 354 916 B1**

(12) **EUROPEAN PATENT SPECIFICATION**

(45) Date of publication of patent specification : **30.09.92 Bulletin 92/40**

(51) Int. Cl.$^5$ : **A61M 1/36,** C12N 11/10, C12Q 1/24, C12N 9/88

(21) Application number : **88903515.0**

(22) Date of filing : **07.03.88**

(86) International application number : **PCT/US88/00699**

(87) International publication number : **WO 88/06899 22.09.88 Gazette 88/21**

# (54) NEUTRALIZATION OF HEPARIN.

(30) Priority : **10.03.87 US 24175**

(43) Date of publication of application : **21.02.90 Bulletin 90/08**

(45) Publication of the grant of the patent : **30.09.92 Bulletin 92/40**

(84) Designated Contracting States : **AT BE CH DE FR GB IT LI LU NL SE**

(56) References cited :
**US-A- 4 373 023**
**Polym. Mater. Sci. Eng., volume 51, 1984, H. Bernstein et al.: "Design of an immobilized enzyme reactor for removing heparinat the termination of extracorporeal therapy", pages 204-207 see page 204, paragraph " Introduction"**

(73) Proprietor : **MASSACHUSETTS INSTITUTE OF TECHNOLOGY**
**77 Massachusetts Avenue**
**Cambridge, MA 02139 (US)**

(72) Inventor : **HEFT, Robert, A.**
**4855 Cote St. Luc Road, Abt. 311, Montreal, Quebec H3W 2H5 (CA)**
Inventor : **COMFORT, Ann, Reese**
**450 Memoral Drive, E111**
**Cambridge, MA 02139 (US)**
Inventor : **LANGER, Robert, S.**
**46 Greenville Street**
**Somerville, MA 02143 (US)**

(74) Representative : **Holdcroft, James Gerald, Dr.**
**Graham Watt & Co., Riverhead**
**Sevenoaks, Kent TN13 2BN (GB)**

EP 0 354 916 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Background of the Invention

This invention is generally in the area of devices for neutralizing heparin and, in particular, devices using heparinase immobilized on high surface area substrates.

Extracorporeal systems perfused with whole blood have been an effective component in the treatment of kidney, heart and lung dysfunction for many years. Unfortunately, the artificial surfaces of machines such as dialysis units and oxygenerators potentiate thrombi and emboli formation. Physicians must therefore rely on systemic heparinization to provide blood compatibility.

Unfortunately, heparin, a mucopolysaccharide consisting of alternating D-glucosamine and D-glucuronic acid subunits and having a molecular weight between approximately 6,000 and 20,000, leads to hemorrhagic complications in many patients. Despite efforts to improve anticoagulation techniques, many patients develop disabling complications when such devices are used. Of the approximately 20,000,000 extracorporeal procedures performed yearly, from 8 to 33% of the patients develop coagulation abnormalities, some of which are life threatening. With increased use of the relatively new membrane oxygenators one can expect longer continuous perfusion times and, in conjunction, the aggravation of these heparin induced problems.Efforts to inject the heparin locally or to otherwise immobilize or remove the heparin have either not been successful in preventing blood clots or have been unable to work in conjunction with high blood flow rates.

Incorporation of a blood filter capable of removing or neutralizing heparin would enable anticoagulation of the extracorporeal circuit while limiting systemic exposure to heparin. The availability of this type of device might allow the use of artificial organs or filters in patients who previously would have been subject to too great of a risk.

U.S. Patent No. 4,373,023 to Robert S. Langer et al teaches using immobilized heparinase to degrade and neutralize heparin in blood. The examples demonstrate that the heparinase can be effectively bound to agarose beads and reacted with the heparin. Unfortunately, when this embodiment was tried on blood at clinical flow rates, the agarose beads packed and the device became unusable. The device described was also useful only in series with other blood treatment devices, not being incorporable into pre-existing devices used for blood treatment.

Another such device is taught by German Offenlegungsschrift DE 3406562 Al to Fraunhofer - Gesellschaft zur Forderung der angewandten Forschung. Heparin degrading enzymes are chemically bound to one side of a membrane having a pare size allowing molecules of less than 25,000 to 30,000 molecular weight to pass through. The apparatus is designed to inactivate that heparin in the bloodstream which is able to pass through the membrane. Unfortunately, the device is unable to provide the high rates of heparin conversion desired clinically due to the requirement that the heparin diffuse through a porous membrane before contacting the enzyme. This limitation is particularly severe considering the high flow rates and thus the short residence time of the heparin in the devices.

It is, therefore, an object of the present invention to provide a device for removal of clinical levels of heparin from the effluent of an extracorporeal device.

It is a further object of the present invention to provide such a device utilizing heparin degrading or neutralizing compounds useful for on-line removal of heparin wherein the compounds are immobilized on a support material which is hemocompatible, mechanically stable, and has structural integrity.

It is a still further object of the present invention to provide a multipurpose device capable of blood treatment as well as heparin removal.

### Summary of the Invention

The present invention eliminates systemic heparin or low molecular weight heparin derivatives by a blood filter containing an immobilized enzyme or other heparin degrading or neutralizing compound is constructed for use at the effluent of an extracorporeal device.

The blood filter must meet specific stringent guidelines for clinical use. The first consideration is biocompatibility of the entire system. The device must not cause hemolysis, platelet aggregation, leukocytopenia, antibody formation, nor release toxic byproducts. The removal system must be compatible with clinical blood flow rates and have an operable life greater than the required perfusion time.

Compounds which are useful in the device of the present invention include enzymes such as heparinase, glucuronate-reductase, aldose-reductase, beta-glucuronidase, O-sulfatase, and N-sulfatase.
Testing has shown that heparinase is neither antigenic nor toxic nor does it produce an antigenic response or release toxic byproducts. Other compounds such as protamine sulfate could be similarly effective using the

appropriate reactor geometry.

A useful system must remove clinical levels of heparin at variable flow rates, up to 4 liters blood/min for an adult to as low as 50 ml blood/min for an infant with perfusion times varying from hours up to between two and ten days for neonatal respiratory failure. At the same time, for some applications, the filter volume must be minimized to allow priming of the circuit with the patient's own blood supply. Finally, the system must be easily operable by the hospital personnel.

Specific embodiments employing heparinase bound by tresyl chloride or CNBr techniques to reconstituted cellulose hollow fiber devices of the type used for kidney dialysis are disclosed. Methods for optimizing the binding and retention of activity of the bound enzyme are also disclosed. In addition to selection and modification of the support and chemical binding technique, the enzyme itself can be modified by genetic engineering to increase the number of binding sites (for example, the number of lysine residues in heparinase). Included are single or multistage devices suitable for heparin removal alone or in combination with dialysis or oxygenation. Also disclosed are analogous multiple membrane sheet devices.

## Brief Description of the Drawings

Fig. 1 is a Lineweaver-Burke plot of the inverse of the reaction rate ($1/V_o$, hr/mg heparinase) versus the inverse of the amount of substrate reacted ($1/S_o$, ml/mg heparin) as a function of flow rate (ml/min).

Fig. 2 is a graph of normalized enzymatic activity versus time (hours) in an aqueous system at 37°C and pH 7.4, demonstrating enzyme stability.

Fig. 3A is a plan view of a device according to the present invention containing heparin immobilized inside hollow fibers of a typical dialysis reactor.

Fig. 3B is a plan view of the device of Fig. 3A without the dialysis jacket, having a greater number of fibers for use with open heart surgery.

Fig. 4 is a plan view of a two stage device according to the present invention wherein only the hollow fibers at the outflow end of a kidney . dialysis device contain immobilized heparinase.

Fig. 5 is a plan view of a variation of the device of Fig. 4 wherein the uncoated hollow fibers are separated from the hollow fibers containing immobilized heparinase by a fluid impervious barrier.

Fig. 6 is a perspective view of a device according to the present invention wherein heparinase is immobilized on multiple layers of membrane sheets.

## Detailed Description of the Invention

The present invention immobilizes a heparin neutralizing or degrading compound, such as heparinase, on a hemocompatible support having sufficient surface area, binding capacity, and structural integrity to allow the reaction of the compound with clinical levels of heparin in an extracorporeal circuit containing blood circulating at flow rates ranging from 50 ml/min up to four 1/min.

The selection of the support is dependent on the following criteria:

The support material must be hemocompatible, i.e., it must not release toxic components into the bloodstream, it must not induce an immunological or thrombogenic response, and it must not react with any of the blood components.

The support material must have sufficient mechanical stability and structural integrity to withstand coupling of the heparin degrading compound, storage, and circulation of the blood at flow rates of between approximately 50 ml/min and 4,000 ml/min.

The support material must be able to bind the heparin degrading compound at sufficient levels and with sufficient retention of activity so that clinical values of heparin can be removed from the circulating blood.

Suggested materials include agarose, cross linked dextran, polyhydroxyl ethyl methacrylate, polyacrylamide, cellulose, and derivatives or combinations thereof, in the form of hollow fibers, as required to obtain sufficient surface area. Suitable devices made with multi-layer hollow fibers are commercially available for use as kidney dialysis units.

The method used to immobilize the compound requires the creation of a strong bond between the support and the compound. In the preferred embodiment, the compound is an enzyme which degrades heparin or low molecular weight derivatives of heparin such as heparinase (EC-4.2.2.7). Other heparin degrading enzymes include glucuronate-reductase (EC-1.1.1.19), O-sulfatase, N-sulfatase, beta-glucuronidase (EC-3.2.1.31), and aldose-reductase (EC-1.1.1.21). Protamine sulfate, which complexes with heparin to neutralize its anticoagulant activity, may also be utilized in some clinical situations. The criteria to determine the appropriate coupling method are: minimization of enzyme leakage from the support, maintenance of the thermal stability of the enzyme, and retention of the optimum amount of heparinase activity. The technique must also not cause a deterioration in the support material or the production of reactive groups on the support which would bind blood components in vivo.

Possible coupling methods and supports are listed in Table 1, along with the uptake (percentage of protein bound to support relative to the amount of protein available in solution), yield (percentage of bound enzyme having activity), and activity (overall amount of active enzyme which is bound to the support, equal to uptake multiplied by yield). Other coupling methods and supports which may be utilized in the present invention will be obvious to those skilled in the art from the detailed description of the present invention.

TABLE 1: SUMMARY OF RESULTS FROM COUPLING STUDIES

| Support | Linking Arm | Activation | Uptake | Activity | Yield |
|---|---|---|---|---|---|
| Avical | | CNBr | | | 0% |
| Dextran | | CNBr | | | 0% |
| Cellulose dialyzer fibers | | CNBr | 90% | 3.6% | 4% |
| Cellulose dialyzer fibers | | cyanuric chloride | 98% | 0% | 0% |
| Cellulose dialyzer fibers | glycine | Woodward's K | 86% | 0.2% | 0.2% |
| | lysine | | 76% | 0.2% | 0.3% |
| | mABA | | 87% | 0.1% | 0.1% |
| | histidine | | 71% | 0.2% | 0.3% |
| | tyrosine | | 86% | 0.2% | 0.2% |
| | alanine | | 83% | 0.2% | 0.2% |
| | arginine | | 82% | 0.2% | 0.2% |
| | glutamic acid | | 33% | 0.2% | 0.6% |
| | B-alanine | | 81% | 0.2% | 0.2% |
| | pABA | | 41% | 2.9% | 7.1% |
| | hexamethylene diamine/ succinic acid | | 38% | 3.3% | 8.7% |
| | chloraoacetyl chloride | | 87% | 0.2% | 0.2% |
| | sodium periodate | | | 4.3% | |
| Sepharose 4B | | CNBr | 97% | 91% | 94% |
| Cellulose dialyzer fibers | sebacyl chloride | Woodward's K | | | 0% |
| Carboxymethylene Sephadex | Woodward's K | 90% | 4% | 4.4% | |
| Carboxymethyl cellulose hydrazide | | | 33% | 1.1% | 3.2% |
| Chloracetyl cellulose | | | 14% | 0.6% | 4.2% |
| Bromoacetyl cellulose | | | 65% | 0.2% | 0.4% |
| Iodoacetyl cellulose | | | 52% | 0.1% | 0.2% |
| Cellulose carbonate | | | 72% | 1.0% | 1.4% |
| Cellulose acetate dialyzer fiber (hydrolyzed) | | CNBr | | | |
| Enzacryl AA | | | 88% | 0.4% | 0.5% |
| Enzacryl AH | | | 40% | 3.8% | 9.5% |
| Support | Linking Arm | Activation | Uptake | Activity | Yield |
| Enzacryl poly-thiolactone | | | 83% | 0.1% | 0.1% |

| | | | | |
|---|---|---|---|---|
| Acrylamide | (polymerization) | 90% | 36% | 40% |
| PMA-$CO_2H$ | EDC | | 0.01% | |
| PMA | Woodward's K | 25% | 0% | 0% |
| PMA-CONH-$(CH_2)_2OH$ | CNBr | | 0% | |
| PMA-BSA | $(CH_2)_6(CHO)_2$ | | 0% | |
| PMA-$(CH_2)_2NH_2$ | $(CH_2)_6(CHO)_2$ | | 0% | |
| PMA-$(CH_2)_6NH_2$ | $(CH_2)_6(CHO)_2$ | | 0% | |
| Maleic Anhydride Ethylene | Anhydride | | 0% | |
| Polyhema | CNBr | 4% | | 0% |
| Polyhema | Sabacic chloride/ Woodward's K | | | 0% |
| Dacron | $(OEt_3)NH_2(CH_2Ch_2OHSi$ (silane) | | 8.6% | 80% |
| Dacron-$NH_2$ | $(CH_2)_6(CNO)_2$ | | 0% | |
| Dacron-$CO_2H$ | Woodward's K | | 0.2% | |
| Silicone-$NH_2$ | $(CH_2)_6(CNO)_2$ | | 0% | |
| Nylon acrylamide | (polymerization) | | | 0% |
| Polyvinyl alcohol | CNBr | | | 0% |
| Polyvinyl acetate (hydrolyzed) | CNBr | | | 0% |
| Polyurethane | (polymerization) | 90% | 0.1% | 0.1% |
| Polyurethane polyvinyl alcohol | CNBr | | | 0% |
| Polyurethane CM cellulose | Woodward's K | 77% | 1.2% | 2.6% |
| Oxirone acrylic beads | | 18% | 1.1% | 6.1% |
| Acrylic annhydride | | | | 0.3% |
| Sepharose 6B-CL | Tresyl chloride | 97% | 39% | 40% |
| Cellulose | Tresyl chloride | 85% | 42% | 50% |

The presently preferred method of coupling heparinase to a support is tresyl chloride activation. Cyanogen bromide activation, periodination, and carbodimidazole activation are also preferred under some conditions.

Tresyl chloride activation has advantages over CNBr activation, the method used in most reports on immobilization of enzymes in similar devices. Tresyl chloride is an activating agent which creates a stable secondary amine bond. Reactive sulfonyl chlorides are used to form sulfonyl derivatives of the resin which then act as good leaving groups. This step allows nucleophilic attack of primary amines and thiols directly on the alcoholic carbon of the resin to form secondary amine groups.

The activation and coupling using tresyl chloride is diagrammed as follows:

$$=|-CH_2\ OH + ClSO_2\ CH_2\ CF_3 \Longrightarrow \qquad [1]$$

support tresyl chloride

$$=|-CH_2\ OSO_2\ CH_2\ CF_3$$

tresylate

$$=|-CH_2\ OSO_2\ CH_2\ CF_3 + H_2N\text{-}L \Longrightarrow \qquad [2]$$

1° amine-ligand

$$=|-CH_2\text{-}NH\text{-}L + HOSO_2CH_2CF_3$$

2° amine-ligand

Cyanogen bromide activation uses a highly toxic reagent to create an isourea bond that is only moderately stable in protein or amine solutions. Using this method, small quantities of heparinase may leach from the filter into the patient's bloodstream. Should this happen, the patient's immune system may be stimulated, although

studies to date demonstrate that this does not appear to be the case.

The cyanogen bromide coupling method is diagrammed as follows: (where L = ligand) -

Activation:

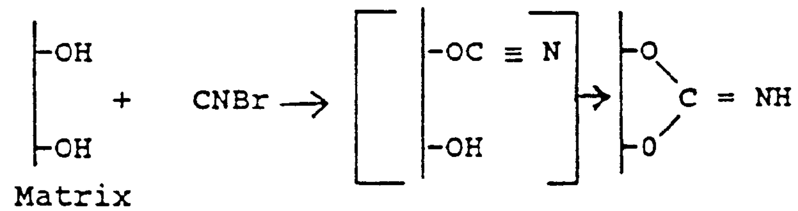

Matrix

Coupling:

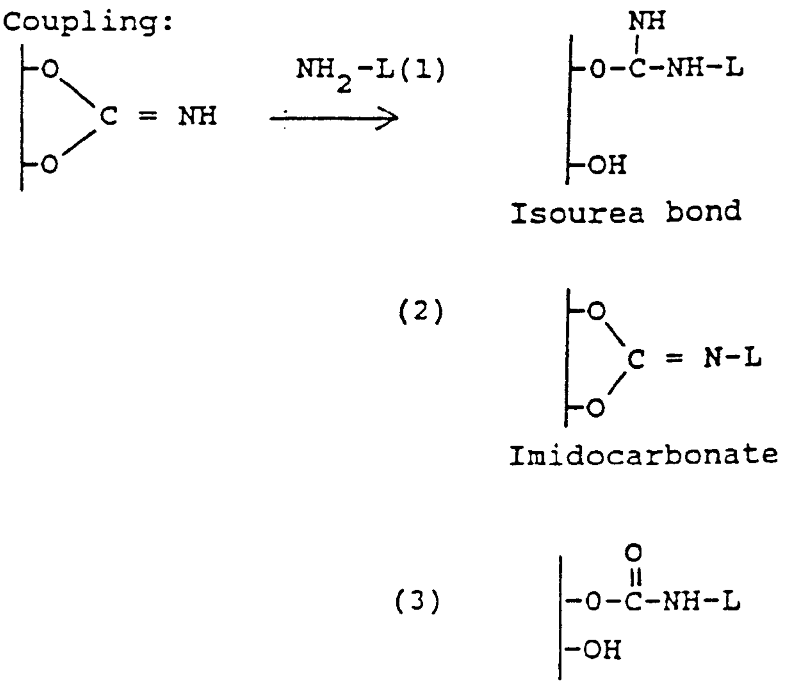


Isourea bond

(2)

Imidocarbonate derivative

(3)

Substituted carbamate

Another criteria is the retention of activity over time. Using tresyl-chloride activated 6% crosslinked agarose beads (Pharmacia Fine Chemical Co., Piscataway, NJ) and cellulose (Sigmacell, type 50), (Sigma Chemical Co., St. Louis, MO), initial coupling studies show that 85 to 97% of all protein binds and that 33 to 50% of the bound enzyme is enzymatically active on the two support materials. Four different samples of cellulose-heparinase were used to test thermal stability of immobilized enzyme at 37°C in 0.725 M Na Phosphate, 0.125 M NaCl at pH 7.0 (isotonic phosphate buffered saline). This study suggested a half-life of 35 hours for tresyl chloride immobilized enzyme, which is not significantly different for that of CNBr-activated agarose.

A third method producing a linkage between an enzyme and an insoluble polysaccharide support is carbodiimidazole activation, described by M.T.W. Hearn et al., in J. Chromat. 185, 463 (1979); R.S. Chapman et al.,in Clinica Chimica Acta. 118, 129 (1982); G.S. Bethel et al., in J. Chromat. 219, 353 (1981); and G.S. Bethel et al., in J. Chromat. 219, 361 (1981), which forms urethane esters with no net charge at the polymer backbone. A lack of charge may be important in the reduction of non-specific binding of blood components. Coupling can proceed in aqueous or organic solvents. Although activated carbamate is relatively stable toward water, over time, all of the unused carbamate hydrolyzes to the original hydroxyl. The urethane bond which is produced is reported to be quite stable.

The carbodiimidazole coupling method is diagrammed as follows:

Activation:

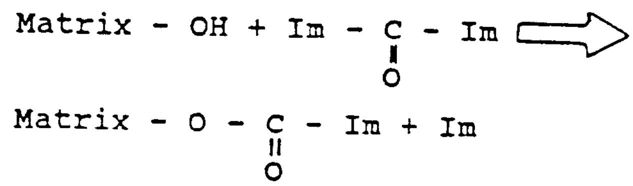

carbamate activated matrix

Coupling:

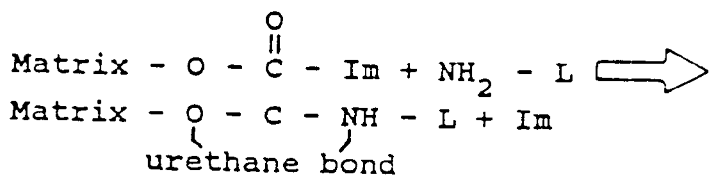


where Im = imidazole, 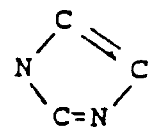 and

L = ligand

Still another method for immobilizing enzyme to a support is periodination, described by C.J. Sanderson et al., in Immunology, 20, 1061 (1971) and J. Turkova et al., in Collection Czechoslova Chem. Commun. 44, 3411 (1979) which uses aqueous solvents to form carbinolamines, yielding alkylamines upon reduction of the support. The conditions for reaction are mild and have the advantage of using aqueous solvents. Experiments suggest that there are fewer non-specific binding effects with this procedure than with CNBr activation.

The periodination coupling method is diagrammed as follows:

Activation:

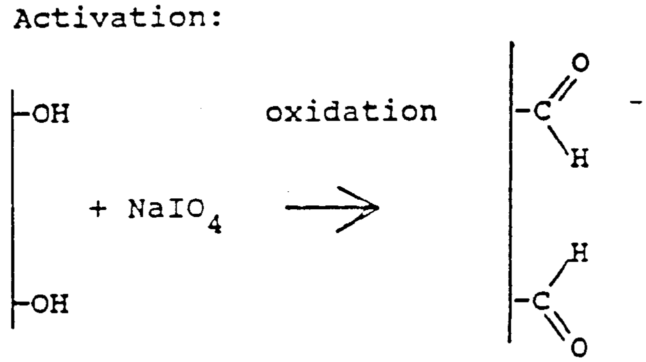


Matrix

Coupling:

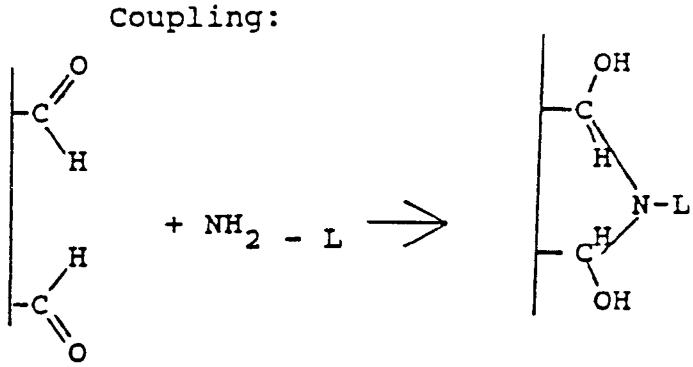


Reduction:

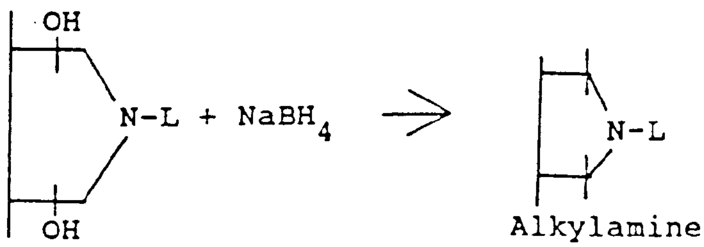


Alkylamine

where L = ligand

Activation:

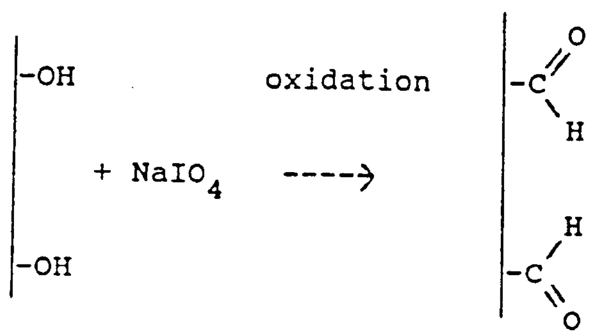


Matrix

Coupling:

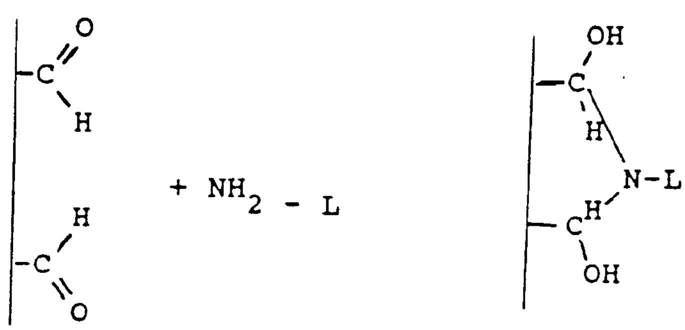

Reduction:

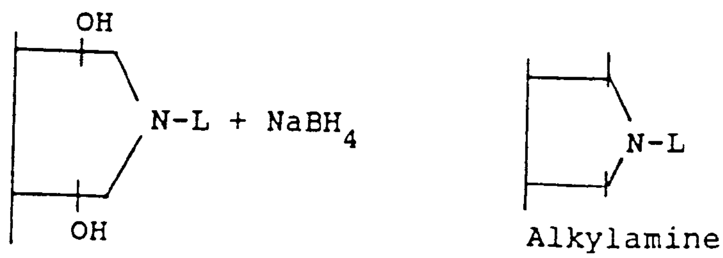


Alkylamine

where L = ligand

The strength of the bond between the enzyme and the support limits the maximum allowable flow rate due to the increased likelihood of enzyme shearing from the carrier matrix. Crosslinking of the enzyme is sometimes effective in decreasing leakage. However, the standard procedure for stabilizing the isourea bond formed by CNBr activation with glutaraldehyde inactivates heparinase.

In a novel approach to increasing the percent enzyme bound to the support, as well as to increasing the percent retention of activity, the number of binding sites on the enzyme are increased. In the case of heparinase, the enzyme is usually bound to the support through the lysine residues. Using methods known to those skilled in the art of molecular genetics and protein chemistry, one can increase the number of lysine residues on the protein. In the preferred method, the gene encoding heparinase is cloned and a nucleotide sequence added which attaches a "tail" of lysine residues to the enzyme. Since lysine residues are located in the active site of the enzyme and therefore play an important role in enzyme activity, this increases the retention of enzyme activity following immobilization on the support. The enzyme may also be modified in the same manner to increase the specific activity of the enzyme.

Another factor limiting flow rate is the stablity of the underlying substrate when exposed to the high flow rates used in extracorporeal blood circuits. In studies of heparinase bound by CNBr activation to 4% agarose beads, in vivo flow rates of 250 ml/min caused the agarose beads to fracture. Crosslinking and increasing the percentage of agarose increases the bed strength. Unfortunately, 10% crosslinked agarose does not show any ability to degrade heparin in vivo. Flow rates of one liter/min or greater are not possible with 8% crosslinked agarose beads.

The degree of enzyme leakage, the thermal stability and the optimal conditions for activity retention with tresyl-chloride, cyanogen bromide activation, and other chemistries, upon immobilization are measured as follows.

To determine the chemical strength of the bond between the support and the enzyme, it is necessary to measure minute concentrations of proteins in buffer and in whole blood. Radiolabeling with $I^{125}$ which binds to the ring structure of tyrosine amino acids, is used to label all proteins immobilized to the support. Following dilution of the labelled sample with unlabeled heparinase, the enzyme is bound to supports via the appropriate coupling method. Supernatants are checked for radioactivity as a function of time and temperature of incubation. In this way, the relative strength of the heparinase-support linkage undergoing mild agitation can be determined. Whole blood studies are used to examine the shearing effects on bound heparinase in various feasible reactor geometries at physiological flow conditions.

The stability of the enzyme against leaching is one of the criteria in choosing the optimal coupling method. Binding of the tresyl-chloride and CNBr activated supports can be compared using leakage of labeled [$I^{125}$] albumin. CNBr activated agarose shows ten times greater loss of the immobilized protein in 0.2 M Tris, pH 7.8 at 37°C than the tresyl-chloride activated agarose over 48 hours.

Loss of enzymatic activity occurs during prolonged exposure of the enzyme to body conditions of 37°C, pH 7.4 and biological agents. Heparinase is very sensitive to temperature increases and to extreme pH values. The best fitting line for free and immobilized heparinase in activation experimental data is an exponential decay of activity as a function of time:

$$E = E_o * \exp(-k_d*t),$$

where E is the enzymatic activity per volume of enzyme,

$k_d$ is the rate of decay, and

t is the time since the initial activity of $E_o$.

The process of inactivation is usually irreversible and results from conformational changes induced by heat,

pH and chemical agents. Heating accelerates the process of enzyme unfolding, pH alters the charges of the various ionizable groups present in the enzyme and chemical agents irreversibly bind to the enzyme or degrade it. Since the loss of enzymatic activity must be considered in the loading of enzyme into the system, the loss of thermal stability during the immobilization step is a constraint in choosing the optimal coupling method.

The final analysis of the coupling method involves the optimization of activity retention determined as a function of three parameters: pH of the coupling solution, the enzyme loading per volume of support, and the number of activated groups introduced per volume of support. Preliminary testing of coupling in a phosphate buffer at 4°C with a contacting time of 20 to 24 hours shows an optimal pH of 8.2 for tresyl chloride activation.

It is necessary to optimize the pH for the greatest retention of bound enzymatic activity. Heparinase activity rapidly degrades a pH greater than approximately pH 8. This optimization is a function of the microenvironment which is not necessarily equivalent to the bulk solution. The optimal pH should be irrespective of the enzyme loading or the number of tresylate groups per volume of support. Varying the enzyme loading and the degree of activation of the support allows the optimization of the activity retention once the enzyme is bound. The retention determines the amount of enzyme required per volume of support and per reactor system.

The half-lives of the immobilized enzyme in buffer and in whole blood are measured by incubating a sample at the temperature of interest (for example, 37°C) in the appropriate medium. The activity of the support as it degrades heparin is measured as a function of time. In buffer, the assay is an ultraviolet assay described by A. Linker and P. Hovingh in Bioch. 11, 563 (1972). In whole blood, clotting assays are used: factor Xa, APTT, thrombin-antithrombin III, as described by E.T. Yin et al., in J. Lab. Clin. Med. 81, 298 (1973); J.W. Estes in Current Therapeut. Res. 18, 58 (1975); L.H. Lam in Biochem. Biophys. Res. Commun. 69, 570 (1976); and L.B. Jacques in Pharmacol. Reviews, 31, 99 (1980). The optimal immobilization technique ideally should not decrease the thermal stability of bound heparinase at 37°C.

Heparinase can be immobilized on supports with varying degrees of activity retention. High yield of enzymatic activity is found using agarose (Pharmacia Fine Chemical Co, Piscataway, NJ) with cyanogen bromide as the activating agent. This hydrophilic beaded gel is composed of spherical particles with diameters in the swollen state of 60 to 140 microns. The gel is 5% agarose and 96% water with a porosity of 96%, has a tortuosity of 1, and a molecular weight cut off of 20 x $10^6$ daltons. The highly porous nature allows heparin to diffuse easily within the support.

The mechanical strength of agarose beads can be increased by either increasing the percentage of agarose or crosslinking the beads with either epichlorohydrin or 2,3 dibromopropanol, using the method of J. Parath et al. in J. Chromat 60, 167 (1971). However, a fifty percent increase in agarose leads to a two to four fold increase in the maximum operating pressure. There are also problems with fracturing of the beads at higher flow rates, as previously discussed.

Cellulose resins are preferred over the agarose supports. Cellulose acetate has been successfully used in extracorporeal devices as hollow fiber dialyzers. Cellulose contains hydrozyl functionalities which can be activated with tresyl chloride, cyanogen bromide, carbodiimidazole or periodination. This resin, structurally stable at 37°C, also has mechanical characteristics which enable its use in filters even at high flow rates. A typical hemodialyzer can withstand a transmembrane pressure of 400 mHg before damage to the filter structure occurs.

An agarose reactor in series with a hemodialyzer degrades 40% of the heparin on a single pass at a flow rate of 250 ml/min. However, after 60 minutes the white blood count is typically decreased by at least 10% and the platelets are reduced by 50%. White and red thrombi also form within the filter although there is no hemolysis. It appears that either the agarose may have hemocompatibility problems due to platelet aggregation or that the heparin removal stimulates the clotting cascade. In contrast, cellulose is used in a number of blood filtering devices and potentiates known whole blood changes without significant complications to the patient.

Between approximately 33-49% heparinase activity has been retained using cellulose particles (Sigmacell, type 50) with tresyl chloride activation. Furthermore, the thermal stability of heparinase-cellulose is equal to that of heparinase-agarose at 37°C. These factors indicate that practical and thermally stable coupling of heparinase to cellulose is possible.

Cellulose is manufactured in many configurations including particulates, membranes, and fibers. The form which avoids the compressibility of cellulose is the hollow fiber unit typically used for ultrafiltration purposes.

The biocompatibility, the mechanical strength, and the structural integrity of the material must be reevaluated after binding of the enzyme. To study biocompatibility, treated and untreated materials should first be checked in vitro for any hemolysis, leukocytopenia, platelet aggregation and any thrombosis. Cellulose-heparinase and cellulose alone should produce equivalent changes in the levels of formed blood components (red blood cells, white blood cells and platelets) which exit the material. Mechanical strength and structural integrity are then tested. Hemocompatibility and consistent maximum flow rates at given pressures must be maintained.

Support materials other than agarose or cellulose may be utilized. For example, polyurethane is particularly

blood compatible. Some of the current blood filters for clot removal use this material. Polyurethane immobilized heparinase has been prepared by reacting freeze dried purified heparinase with polyethylene glycol (PEG) capped toluene diisocyanate prepolymer. This latter method has not yet demonstrated a good retention of activity by the bound enzyme, however.

The required surface area, reactor geometry, and units of enzyme activity for a particular use can be approximated using the following equations relating to the enzyme kinetics. once a model device is constructed, the device can be optimized empirically by one skilled in the art.

Experimental investigations of an immobilized enzyme-support system must begin with the effective rate and kinetic parameters. Unfortunately, these parameters only fit specific conditions and cannot describe the system in general. The observed kinetic behavior of an immobilized enzyme system can be significantly affected by partition effects and diffusional resistances.

General methods have been developed to treat external diffusion, internal diffusion and the immobilized enzyme kinetics. The immobilized enzyme kinetics obey classical Michaelis-Menten Laws (K.J. Laidler and P. Bonting in The Chemical Kinetics of Enzyme Action, 2nd edition (Oxford University Press, NY 1973)) since product and substrate inhibition are not observed in solution. Since this is an open flow system, even at relatively high concentrations at steady state, the Michaelis-Menten kinetic law adequately describes the classical enzyme mechanism. External diffusion is related to the mass transfer coefficient. This parameter is readily quantified for a variety of geometries and flow rates from heat and mass transfer correlations and can be removed generally from the observed reaction rate by increasing flow rates. Unlike external diffusion, internal diffusion proceeds in parallel with the chemical reaction and is therefore more difficult to remove. The general methods are adaptable to the various possible geometries.

The Michaelis-Menton kinetic laws are applied as follows.

For the kinetically controlled reactions,

$$V_{kin} = V_{max} * S/[K_m(app) + S]$$

where $V_{max}$, a function of the enzyme concentration, is the saturation rate at the fiber surface, S is the substrate concentration in bulk, and $k_m$(app) is the Michaelis constant.

$V_{kin}$ (moles/cm$^2$ sec) is the maximum rate of reaction for a given substrate concentration and is defined as the inherent reaction rate when diffusion of the substrate is infinitely fast.

Two limiting cases are important here. If the level of substrate at the support wall, $S_o$, is much higher than $K_m$, then the rate is a function of enzyme loading and there are no diffusional effects. When $S_o$ is much less than $K_m$, the rate is first order in $S_o$ and related to $V_{max}$ /$K_m$(app). $K_m$(app) is influenced by conformational, environmental and diffusional effects. The theory predicts, for flow through a tube, that $K_m$ (app) will increase with increasing diffusional control according to

$$K_m(app) = K_m + (V_{max}/2 * K_1)$$

where $K_m$ is the diffusion free value of the kinetic parameter. The observed $K_m$(app) then varies with the reciprocal cube root of the linear flow rate in the diffusion limited régime. The reaction rate is also inversely proportional to $K_m$(app) so that with increasing diffusional control (lower flow rate, larger R or L, where R is the radius of the tube and L is the tube length) the reaction rate at low $S_o$ diminishes.

These results are easily related to overall reaction rates and exit concentrations. The rate of product formation, $V_o$, is

$$V_o = 2 * 3.14 * R * L * V$$

and is independent of the flow rate. For the case of diffusion control, the rate of product formation is

$$V_{o,d} = 8.06 * (V_f * D^2 * R^2 * L^2)^{1/3} * S$$

and the exit concentration of substrate where the conversion is determined by product formation divided by the volumetric flow rate is

$$S_{exit} = [1- 2.56 * (D L/(R^2 * V_f))^{2/3}] * S$$

When the reaction is kinetically controlled, the production rate is

$$V_{o,k} = 2 * 3.14 * R * L * V_{max} * S/(K_m + S)$$

and the exit concentration of substrate is

$$S_{exit} = [1 -[2 * L * V_{max} / (R * V_f (K_m + S))]] * S$$

The flow rate dependence varies in a characteristic manner for the particular régime. For example, $S_{exit}$ varies with $V_f^{-2/3}$ if there is diffusion control and $S_{exit}$ varies inversely with $V_f$ if there is kinetic control. This knowledge is important for the discrimination of the inherent kinetic parameters from diffusional effects.

This method is developed from general mass transfer and kinetic principles. The character of the medium, buffer or blood, is included in the original description of the system. The final results, to a first approximation, are independent of the viscosity or the density of the medium. The parameter which governs an analytical treatment is the diffusivity of the substrate in the medium. Generally, it is only necessary to have an order of magnitude assessment of this parameter since man of the graphical techniques involve easily measurable

quantities such as flow rate, reaction rate and substrate concentration to analyze the régime of interest.

Hoewver, it is possible to calculate the predicted diffusivity of a solution in a specific reactor. In the preferred embodiment of the present invention, wherein the enzyme is attached directly to cylindrical fibers, where there are no pores large enough for the enzyme to diffuse into the support material and the enzyme is bound in a unimolecular layer on the tube wall, there will be no internal diffusional effects since these are limited to enzyme bound within a solid matrix. There are diffusional resistances between the bulk solution and the surface of the cellulose fiber. External diffusion encompasses both molecular and convective forces.

There are two limiting regimes which determine the observed rates of reaction: bulk diffusion controlled and catalytically controlled. It is possible to isolate these two limiting cases for use in reactor design. A diffusional boundary layer is assumed to lie adjacent the inner tube wall. The initially flat concentration profile, independent of the radial coordinate, gradually changes to an established profile dependent on the radial coordinate. The thickness of the diffusion layer may be regarded as considerably smaller than the radius of the tube. The width of the diffusion layer depends upon the mass transfer coefficient, which is a measure of the rate of substrate diffusion in a direction perpendicular to the bulk flow. The mass transfer coefficient, averaged over the length of the reactor, varies as the cube root of the flow rate. When the enzyme reaction is so fast that the concentration of substrate at the wall goes to zero:

$$L / 2 * R \text{ less than } N_{Re} * N_{Sc} / 100,$$

where $N_{Re}$ and $N_{Sc}$ are the Reynolds and Schmidt dimensionless numbers, respectively, the product of $N_{Re}$ and $N_{Sc}$ is independent of viscosity and the density of the system is

$$N_{Re} * N_{Sc} = 2 * R * V_f / D$$

Knowing the diffusivity of the substrate in whole blood, it is possible to see if the reactor will be diffusion controlled due to its flow rate and tube radius. As $k_1$ increases, the diffusion layer thins; this occurs at high flow rates. At low flow rates, the diffusion layer is thick and diffusional effects ar observable.

The flux of substrate to the wall surface behaves like a reaction rate in that the molecules must arrive at the wall to be degraded. This flux (moles/cm$^2$ sec) is related to substrate concentrations by

$$J_s = k_1 * (S - S_o)$$

where S is the substrate concentration in the bulk and $S_o$ is that at the support wall. When the enzymatic reaction is very fast, the surface concentration at the wall goes to zero and the maximum rate per area is governed by diffusion as

$$V_d = k_1 * S$$

and the reaction is first order with respect to the substrate concentration.

In order to design a reactor, one first needs the characteristic reaction rate profile for a variety of conditions. Through experimental observation and the utilization of techniques in the previous section, the kinetic parameters can be defined. The variables in the reactor design, then, are space time, substrate inlet concentration and enzyme concentration. Space time is proportional to the reactor volume, $V_R$, and inversely proportional to the volumetric flow rate, F. For steady state operation of a plug flow reactor with laminar flow and isothermal conditions, the design equation is

$$V_R/F = {}_s(-r_{s)}{}^{-1} * dS$$

This design equation or one for the appropriate geometry, including the reaction rate profile, $(-r_s)$, and the thermal inactivation of the enzyme, creates a base for simulation studies to model the performance of any reactor for the range of operating conditions,.tube diameters, substrate and enzyme concentrations. In this way, one can determine the appropriate reactor design for optimal substrate conversion given the operating conditions. The initial react ion rate can be measured from a study of substrate levels and flow rates, as used clinically.

A more reliable linear depiction of initial rate data is the Eadie-Hofstee plot. This plot is a linearized version of the Michaelis-Menten equation

$$V = Vmax^{-(V/S) * K_m}$$

These plots yield straight lines when V obeys Michaelis-Menten kinetics and is not diffusion controlled. To detect deviations the concentration range must be wide. This graph compared to the standard Lineweaver-Burke plot has more pronounced deviations from straight lines when it is diffusion limited.

At this point, the system is tested in circuit with an extracorporeal device. The in vitro studies follow substrate conversion as a function of flow rate, reactor volume, inlet substrate concentration and enzyme concentration. Concurrently, hemocompatibility testing is performed. This includes inlet and outlet evaluation of the formed blood components and their relation to the reactor performance. These performance studies are not expected to mimic exactly the reservoir blood system used in the in vivo cases. The blood viscosity, formed blood components and coagulability characteristics will govern the fluid dynamics and mass transfer in the system and, thus, the overall reactor performance.

The following are embodiments of the present invention utilizing the criteria described in the preceding detailed description for how to select an appropriate support, coupling technique, required enzyme loading and reactor design for an extracorporeal device useful in removing systemic heparin. These embodiments overcome many of the limitations of the devices described in the background of the invention. specifically, they are able to remove clinical levels of heparin at flow rates characteristic of kidney dialysis or open heart surgery, they are not diffusion limited, and they do not exhibit undesirable loss of bound enzyme activity, either due to leaching or inactivation. Further, they are capable of being utilized for multiple purposes, including dialysis and purification, as well as for neutralization of heparin anticoagulant activity.

Selection of support material and reactor geometry:

Cellulose has been selected as the presently preferred support material. Cellulose contains hydroxyl functionalities which can be activated with a number of chemistries to allow covalent binding of proteins to this insoluble support. Regenerated cellulose and cellulose acetate have been used successfully in clinical extracorporeal devices such as hollow fiber and multiple membrane sheet kidney dialyzers. Hollow fibers are the form employed in the claimed invention. These fibers have mechanical characteristics which enable their use in blood filters even at high flow rates of one liter/min through a 150 ml device. Bundles of parallel narrow fibers create an adequate surface area for the enzymatic reaction with the inherent possibility of external diffusional limitations.

The number and length of the fibers, as well as the diameter, are calculated to result in the desired heparin removal while allowing the flow of blood at the desired flow rate and without creating an excessive pressure drop through the device. In general, the commercially available hemodialyzers have a surface area of 1.3 $m^2$. Pediatric hemodialyzers have approximately one-half this surface area. Modified for use with open heart surgery, the surface area would be increased some four to ten fold.

Selection of heparin neutralizing compound:

Heparinase has been selected as the preferred compound for binding to the cellulose support for neutralization of heparinase activity.

Selection of coupling technique:

Tresyl chloride and cyanogen bromide were selected as the coupling techniques. Tresyl chloride gives better loading results on the cellulose support. The following is a comparison of bound enzyme yield for heparinase bound to agarose and regenerated cellulose using both coupling techniques.

| Support | Activation | Yield(%) |
|---|---|---|
| agarose | CNBr | 45 |
| | tresyl chloride | 15 |
| cellulose | CNBr | 0.1 |
| | tresyl chloride | 10-25 |

The physical structure of the material following the activation procedure has been shown to be intact. Qualitative confirmation of a lack of fracturing and pore damage to the support was shown with high power light microscopy. Macroscopically, the pressure, flow rates and ultrafiltration rates are consistent with the original operational characteristics of the cellulose fibers prior to the chemical treatment.

Coupling of enzyme to cellulose hollow fibers.

The commercially available devices, containing fibers of cellulose or cellulose acetate, must be modified when the heparinase is to be immobilized using organic solvents since the epoxy bases holding the hollow fibers are not resistant to organic solvent. The glycerin in the fibers, used to enhance stability of the fibers during storage, must also be removed. Using the presently preferred method of tresyl chloride to bind the heparinase to the hollow fibers, the fibers are flushed repeatedly with acetone to remove all traces of water. The fibers

are then immersed in the solution of tresyl chloride and allowed to react for a time necessary to create the optimum number of active sites for binding the heparinase. This optimum number is a function of the number of lysines (37 in heparinase), the need to adequately hold in the enzyme in the face of the flow rate and the various proteases in the bloodstream and the requirement that the active site of the enzyme not be blocked. This number is approximately 100 tresyl chloride sites for heparinase. The pH of the solution is also crucial to the binding of the heparinase. Heparinase loses activity at a pH greater than 8. Tresyl chloride binds most effectively at a pH of 8 to 9. Since binding may take as long as a day, the reaction is preferrably performed at a pH of 8. Binding may also be accomplished by recirculating the heparinase solution through the fibers but does not produce as homogeneous a result as immersion for a longer period of time. Binding may be followed by crosslinking of the enzyme with glutaraldehyde or other dialdehyde or other crosslinking agent such succinic anhydride. Crosslinking serves to stabilize the enzyme on the support surface but must be limited so as to not block the active site of the enzyme.

Determination of enzyme loading and activity:

The initial reaction rate was studied as a function of flow rate and substrate concentration. At high linear flow rates of 8-10 cm/sec, the observed reaction rate is equal to the intrinsic maximum enzyme reaction rate. The maximum reaction rate is determined by the absolute loading of enzyme on the hollow fiber surface. Conversion of heparin increases linearly with increased enzyme loading. Since the enzyme is too large in molecular weight to diffuse into and through the porous hollow fiber, the amount of enzyme bound to the fiber is limited by the outer surface area. The current Maximum enzyme loading on the internal surface of cellulose fibers is 0.22 mg heparin degraded / hr-$cm^2$[units activity/$cm^2$]. Commercially available heparinase has an initial specific activity of 200 units per mg protein. This activity is observed when 4.5 micrograms of protein are bound to the fibers per $cm^2$ of regenerated cellulose, depending on the source of heparinase, as well as the characteristics of the heparin. As discussed earlier, immobilized heparinase can be used to degrade both heparin and low molecular weight derivatives of heparin.

Combining the maximum enzyme loading, intrinsic kinetic parameters, and diffusivity data, the optimal blood filter for maximum heparin conversions at variable flow rates can be designed.

If the $K_m$(app) is assumed to be 2.5 mg heparin/ml, then the following conversions can be expected at the respective flow rates and with the respective enzyme loadings on an 85 ml, 10,000 fiber, system ($S_o$ = 0.02 mg heparin/ml) in physiological buffer:

| Flow rate (ml/min) | 5000 units enzyme Conversion (%) | 25,000 units enzyme Conversion (%) | Diffusion limited Conversion (%) |
|---|---|---|---|
| 100 | 28 | 80 | 88 |
| 200 | 15 | 55 | 73 |
| 400 | 8 | 33 | 57 |

This postulated $K_m$(app) is more than ten times greater than that observed for the intrinsic constant in an agarose system. The first column of numbers for 5000 units of enzyme corresponds to the current loading of heparinase on the fibers. This loading uses catalytically pure enzyme (200 units activity per mg protein). The 25,000 units of enzyme correspond to an increase in enzyme purity to a level of 1000 units of activity per mg protein (for reference, completely pure heparinase has an activity on the order of 2,000 to 20,000 units per mg of protein). Increasing the purity of the heparinase by a factor of five increases the degradation of heparin per pass through the device. The diffusion limited case is included for comparison. This is the theoretical limit of conversion of heparin in an aqueous system. This limit is dependent on the geometry of the device (radius and length of fibers) and the diffusivity of heparin.

Construction of reactor and performance in vitro:

A simple whole blood system was constructed using 50 fibers with a total internal volume of 0.45 ml. These

fibers were composed of regenerated cellulose (Spectrum Medical, Los Angeles,CA) of 107 microns in radius and 28 cm in length and a volume of approximately 0.6 ml. This bundle of fibers had an enzymatic activity of 21 mg heparin degraded per hour bound covalently to the lumen. A Lineweaver-Burke plot is shown in Figure 1 for this device
. Whole heparinized blood was recirculated through the device. The circuit had a total blood volume of 32 ml. At selected time intervals samples were taken before and after the reactor and examined for clotting time. The activated clotting time test (ACT) is directly correlated with heparin levels in whole blood. The general trend shows a decrease in clotting time over the length of the experiment. This signifies that heparin was degraded in whole blood by the hollow fiber device.

An important aspect of this device is the enzyme's thermal stability with respect to the patient's body temperature of 37°C. The half-life of the enzyme was determined in an aqueous system at 37°C and pH 7.4 . Figure 2 shows the normalized results. The half-life in an aqueous system is 32 hours. This is sufficient for the expected extracorporeal applications of this device.

Preferred embodiments according to the present invention are depicted in Figures 3 to 6.

Figure 3A is a device 10 formed from a commercially available kidney dialysis unit consisting of a cylindrical dialysis jacket 12 having an inlet 14 and an outlet 16 for blood in an extracorporeal circuit (not shown), a bundle 18 of hollow fibers, and an inlet 20 and outlet 22 for dialysate or buffered isotonic solution. The size and configuration of the device 10 are determined for use in conjunction with kidney dialysis. The blood flow rates typical of kidney dialysis are in the range of 200 ml/min.

Figure 3B is a device 30 having an inlet 32 and outlet 34 for blood in an extracorporeal circuit, a bundle 36 of hollow fibers, and a buffered isotonic solution surrounding the bundle 36 within a sealed container 38, replacing the dialysis jacket 12 of device 10.. The size and configuration are determined for use in conjunction with open heart surgery. The primary differences between the device 10 and the device 30 are the sealed container 38 and the larger number of fibers 36, for handling flow rates of approximately 3000 to 4000 ml/min.

In both devices 10, 30 the hollow fiber bundles 18, 36 have been treated to immobilize heparinase within the fibers at a concentration and with a percent retained activity calculated to remove the majority of the heparin contained in the blood passing through the device.

The hollow fibers in the device 30 of Figure 3B may additionally be treated to coat the outside of the fibers to prevent the loss of important electrolytes. The configuration is then of a series of parallel tubes having enzyme immobilized within them.

Figure 4 is a device 40 having a first section 42 of hollow fibers for dialysis and a second section 44 containing immobilized heparinase for removal of heparin. Using this device, the patient can be on dialysis for the average period of four hours, with the heparin's anticoagulant activity being continuously neutralized prior to the blood returning to his body.

Figure 5 is a device 50 having a first section 52 of hollow fibers for dialysis and a second section 54 of hollow fibers containing immobilized heparin wherein the two sections 52,54 are separated by a fluid impervious barrier 56 on the dialysate side of the fibers 52,54. The fluid impervious barrier 56 can be formed from an epoxy resin or other similar materials. This device 50 is particularly useful in situations wherein the enzyme activity is sensitive to the dialysate environment or the dialysis unit length needs to be shortened for efficacy of removal of solutes.

Figure 6 is a device 60 similar to the devices shown in Figures 3 to 5, but formed of two flat end pieces 62,64, a series of flat or pleated membrane sheets 66 for dialysis and/or heparin removal, inlet 68 and outlet 70 for the blood, and inlet and outlet 72 for the dialysate fluid.

The present invention of immobilizing a heparin degrading or neutralizing enzyme or other compound in a device having high surface area, biocompatibility, and mechanical strength for removal of heparin on-line with an extracorporeal circuit has been described with reference to specific embodiments.

## Claims

1. A device for extracorporeal heparin neutralization comprising
   a substance for contacting blood directly which specifically neutralizes the anticoagulant activity of heparin and low molecular weight derivatives of heparin; and,
   a support having sufficient surface area to expose the neutralizing substance to the heparin in the blood at a flow rate of between approximately 50 ml/min and 4000 ml/min and having mechanical stability, structural integrity, and
   hemocompatability under these conditions; and the said support is in the form of a plurality of hollow fibers, which are for example porous.

2. The device according to Claim 1, wherein the support is formed of a material selected from regenerated cellulose, cellulose acetate, agarose, crosslinked dextran, polyhydroxyethyl methacrylate, polyacrylamide, and derivatives thereof.

3. The device according to Claim 1 or Claim 2, wherein the neutralizing substance is selected from enzymes consisting of heparinase, glucuronate-reductase, aldose-reductase, beta-glucuronidase, O-sulfatase, and N-sulfatase.

4. The device according to Claim 3, wherein said neutralizing substance is modified to increase the number of binding sites for the support or the substrate.

5. The device according to Claim 4, wherein said substance is heparinase modified to increase the number of lysine residues present on the enzyme.

6. The device according to any of Claims 1 to 5, wherein the neutralizing substance is immobilized on the support by a bond formed using cyanogen bromide, tresyl chloride or carbodiimidazole.

7. The device according to any of Claims 1 to 6, further comprising, in series with said support, means for the treatment of blood to add oxygen or remove impurities.

8. The device according to Claim 7, further including a fluid impervious barrier separating the support containing the immobilized substance and the means for treating the blood.

**Patentansprüche**

1. Vorrichtung zur extrakorporalen Heparin-Neutralisation umfassend
   - eine Substanz zum direkten Kontakt mit Blut, welches die antikoagulierende Aktivität des Heparins und niedrig-molekularer Derivate des Heparins spezifisch neutralisiert, und
   - einen Träger mit ausreichender Oberfläche, um die neutralisierende Substanz der Einwirkung des Heparins im Blut bei einer Durchflußgeschwindigkeit von annähernd 50 ml/min bis 4000 ml/min auszusetzen,

   der mechanische Stabilität, strukturelle Integrität und Verträglichkeit dem Blut gegenüber unter diesen Bedingungen aufweist und

   in der Form einer Vielzahl von Hohlfasern vorliegt, welche beispielsweise porös sind.

2. Vorrichtung nach Anspruch 1, worin der Träger aus einem Material gebildet ist, das aus regenerierter Cellulose, Celluloseacetat, Agarose, vernetztem Dextran, Polyhydroxyethyl-methacrylat, Polyacrylamid oder deren Derivaten besteht.

3. Vorrichtung nach Anspruch 1 oder Anspruch 2, worin die neutralisierende Substanz aus Enzymen, nämlich Heparinase, Glucuronat-Reductase, Aldose-Reductase, Beta-Glucuronidase, O-Sulfatase oder N-Sulfatase besteht.

4. Vorrichtung nach Anspruch 3, worin die neutralisierende Substanz zur Erhöhung der Zahl der Bindungsstellen an den Träger oder das Substrat in modifizierter Form vorliegt.

5. Vorrichtung nach Anspruch 4, worin die Substanz zur Erhöhung der Zahl der Lysinreste an dem Enzym heparinase-modifiziert vorliegt.

6. Vorrichtung nach einem der Ansprüche 1 bis 5, worin die neutralisierende Substanz durch eine unter Anwendung von Cyanogenbromid, Tresylchlorid oder Carbodiimidazol ausgebildeten Bindung auf dem Träger fixiert ist.

7. Vorrichtung nach einem der Ansprüch 1 bis 6, wobei diese zusätzlich in Serienschaltung mit dem Träger Mittel zur Behandlung des Blutes zur Sauerstoffzugabe oder Entfernung von Verunreinigungen umfaßt.

8. Vorrichtung nach Anspruch 7, weiterhin umfassend eine flüssigkeitsundurchdringliche Barriere, die den Träger mit der darauf fixierten Substanz und die Mittel zur Blutbehandlung voneinander trennt.

## Revendications

1. Dispositif pour la neutralisation extracorporelle de l'héparine comprenant
   une substance destinée à entrer en contact direct avec le sang et qui spécifiquement neutralise l'activité anticoagulante de l'héparine et des dérivés de faible poids moléculaires de l'héparine, et
   un support ayant une surface suffisante pour exposer la substance neutralisante à l'héparine dans le sang à une vitesse de flux entre approximativement 50 ml/min et 4000 ml/min et étant sous ces conditions mécaniquement stable, structurellement intègre et hémocompatible, et ledit support a la forme d'une pluralité de fibres creuses qui sont par exemple poreuses.

2. Dispositif selon la revendication 1, dans lequel le support est formé d'une matière choisie parmi la cellulose régénérée, l'acétate de cellulose, l'agarose, le dextrane réticulé, le méthacrylate de polyhydroxéthyl, le polyacrylamide et les dérivés de ceux-ci.

3. Dispositif selon la revendication 1 ou 2, dans lequel la substance neutralisante est choisie parmi les enzymes consistant de héparinase, de glucuronate-réductase, aldose-réductase, béta-glucuronidase, O-sulfatase et N-sulfatase.

4. Dispositif selon la revendication 3, dans lequel la substance neutralisante est modifiée pour augmenter le nombre des sites de liaison pour le support ou le substrat.

5. Dispositif selon la revendication 4, dans lequel ladite substance est l'héparinase modifié pour augmenter le nombre des résidus de lysine présents sur l'enzyme.

6. Dispositif selon une des revendications 1 à 5, dans lequel la substance neutralisante est immobilisée sur le support par une liaison formée en utilisant du bromure de cyanogène, du chlorure de trésyl ou du carbodiimidazole.

7. Dispositif selon une des revendications 1 à 6 comprenant en outre, en série avec ledit support, un moyen pour le traitement du sang pour ajouter de l'oxygène ou enlever des impuretés.

8. Dispositif selon la revendication 7, comprenant en outre une barrière imperméable aux fluides séparant le support contenant la substance immobilisée et le moyen pour le traitement du sang.

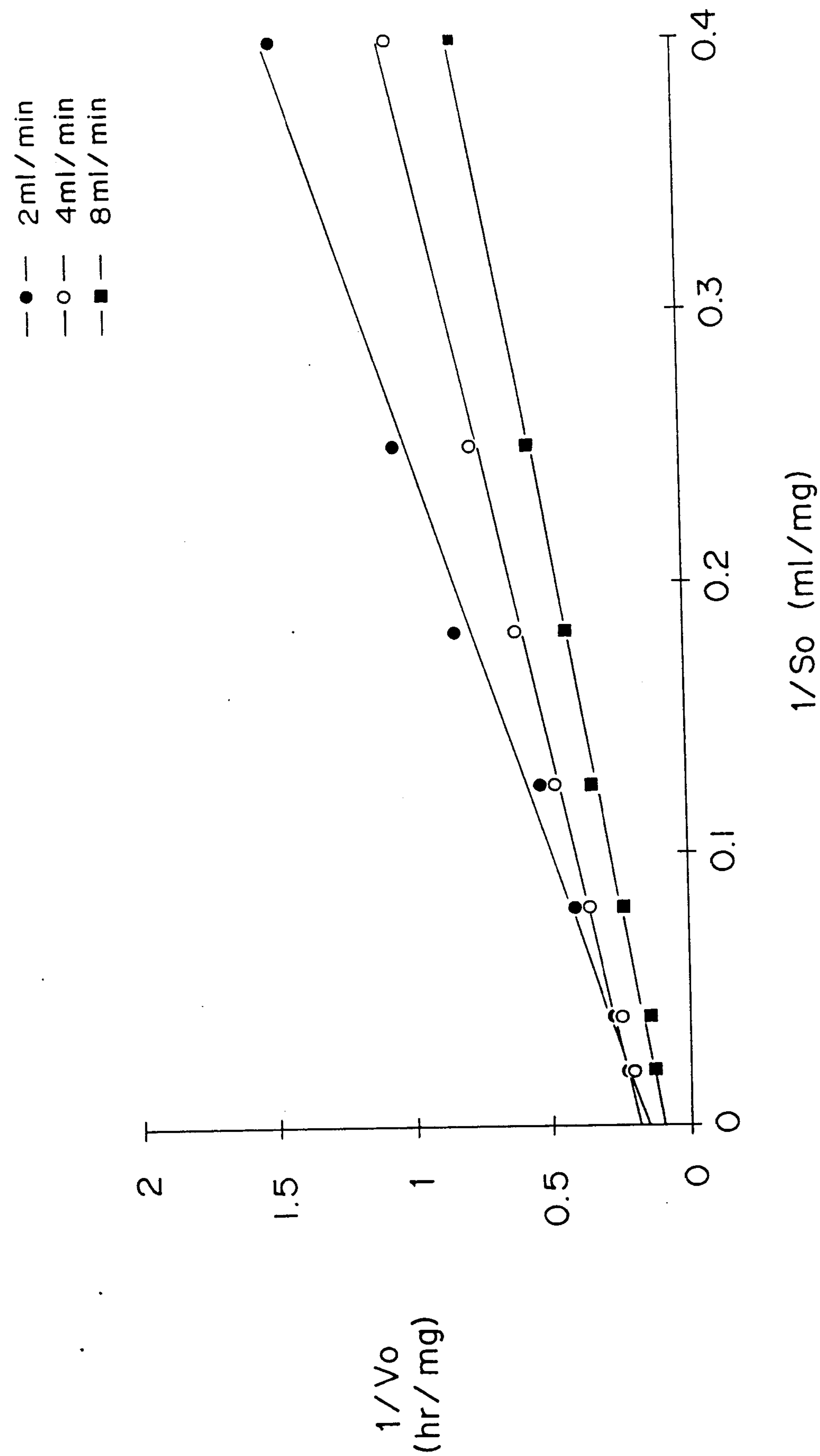


FIGURE 1

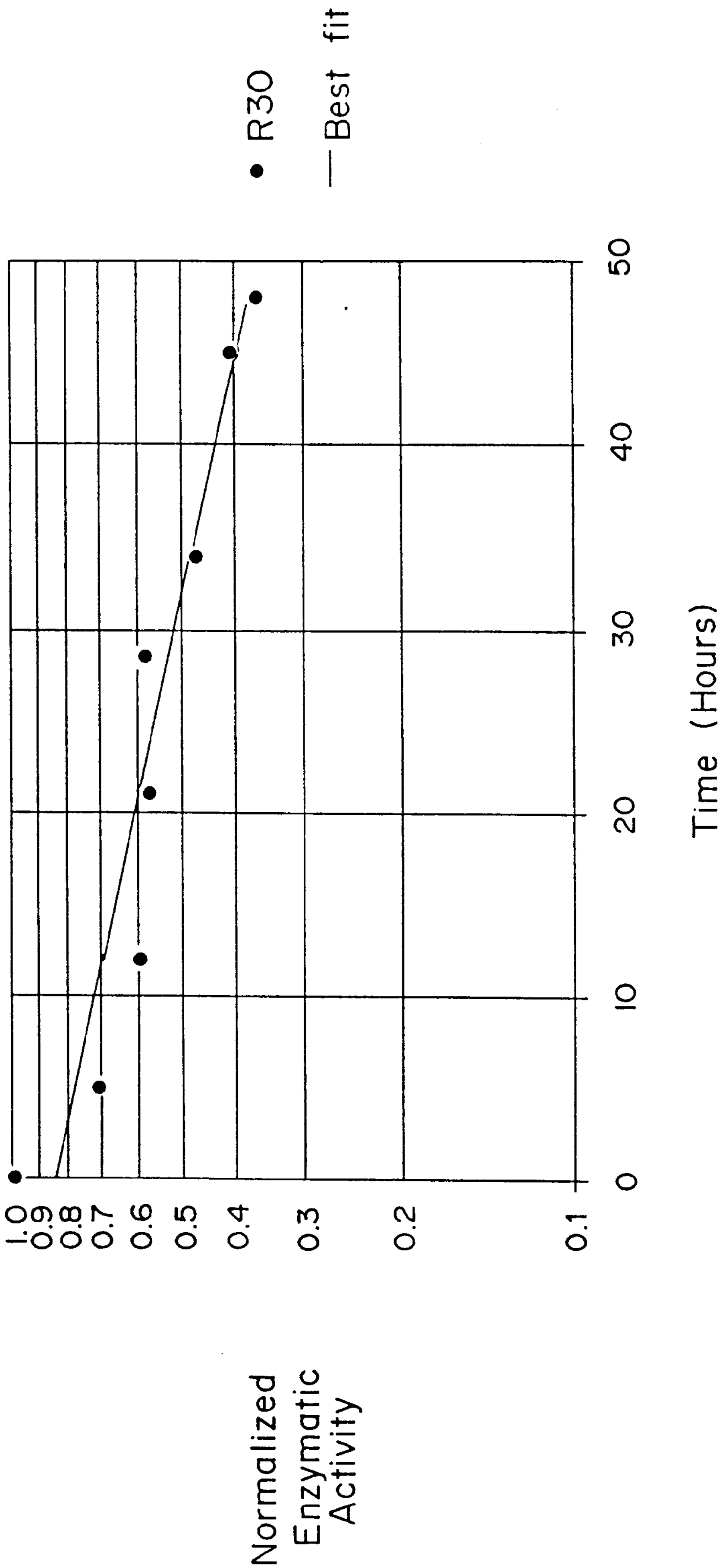

R30
Best fit
Normalized Enzymatic Activity
1.0
0.9
0.8
0.7
0.6
0.5
0.4
0.3
0.2
0.1
0
10
20
30
40
50
Time (Hours)


FIGURE 2

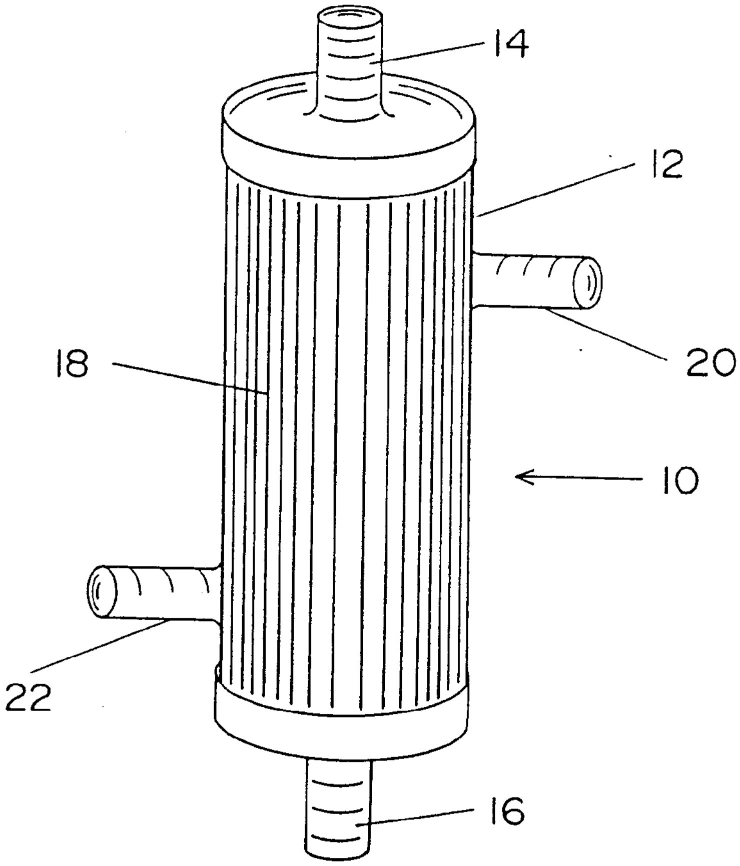


FIGURE 3a

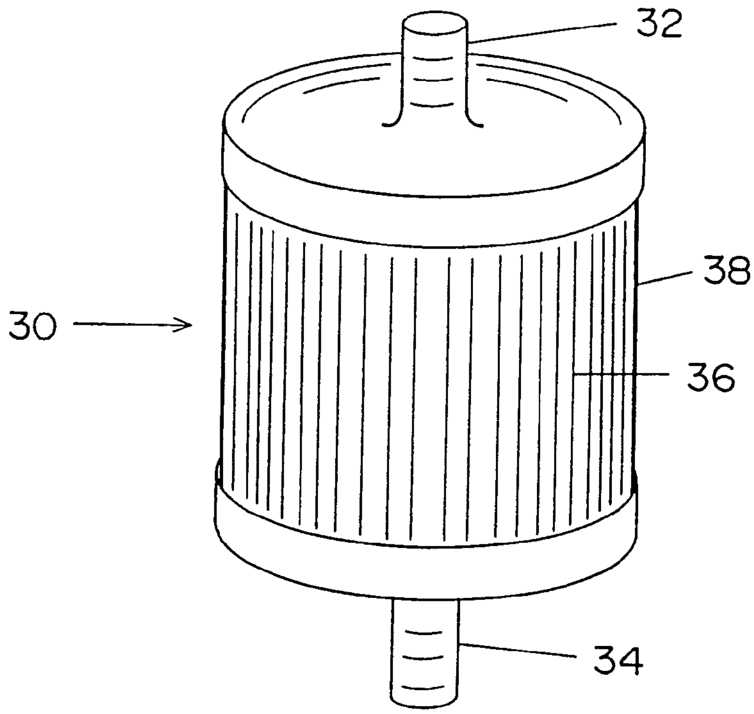


FIGURE 3b

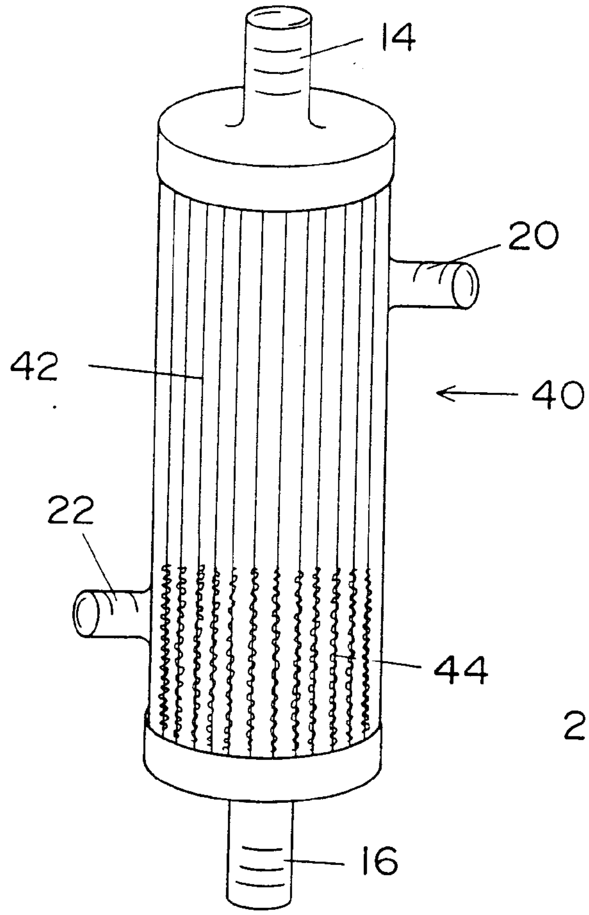


FIGURE 4

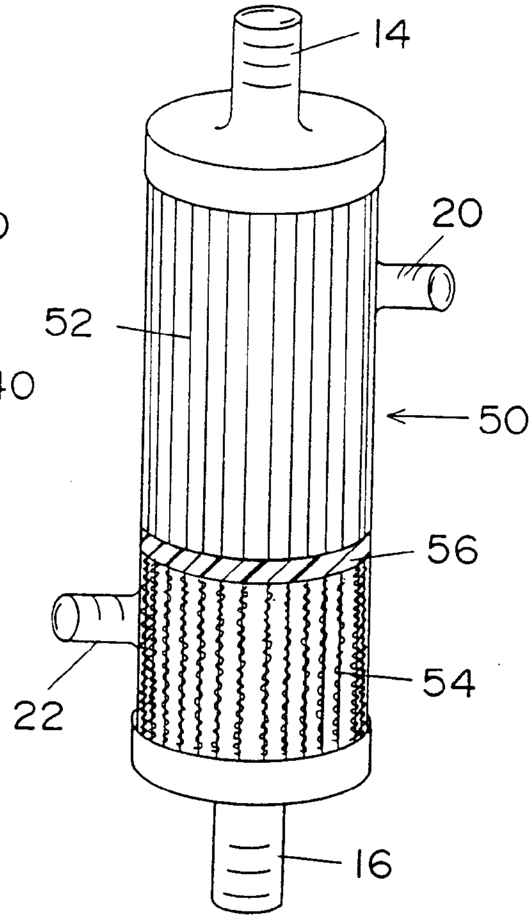


FIGURE 5

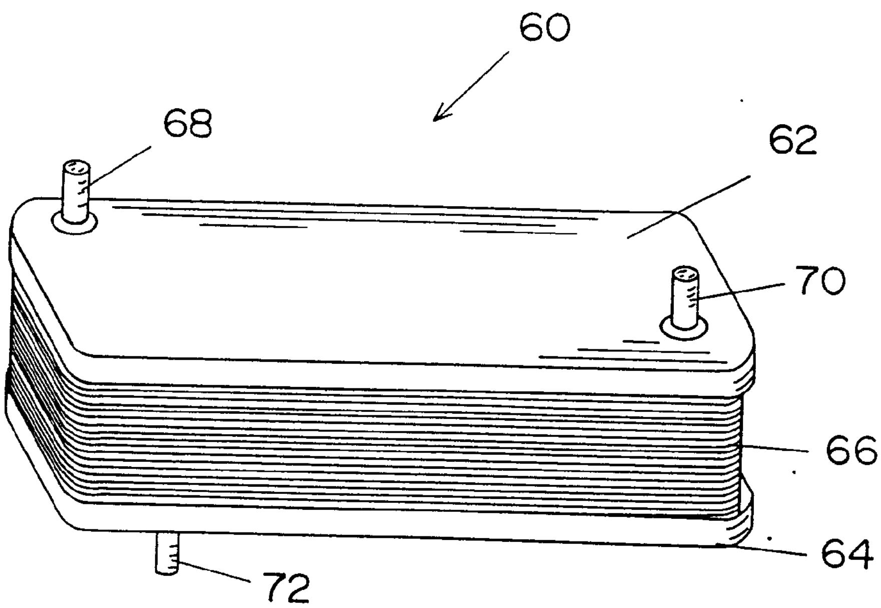


FIGURE 6